# EUROPEAN PATENT APPLICATION

(11) **EP 4 534 056 A1**
(43) Date of publication of application: **09.04.2025**
(21) Application number: 23207752.9
(22) Date of filing: 03.11.2023
(51) Int. Cl.: A61F 13/08, A61F 5/00, A61F 13/12

(54) **COMPRESSION GARMENT ASSEMBLY SET, COMPRESSION GARMENT AND METHOD FOR ASSEMBLING A COMPRESSION GARMENT**

(30) Priority: 02.10.2023 US 202363587286 P
(71) Applicant: Medi USA, L.P., Whitsett, NC 27377 (US)
(72) Inventor: GROLEAU, Bryan, St. Augustine, 32080 (US); LIPSHAW, Moses, Hillsborough, 27278 (US)
(74) Representative: Lindner Blaumeier Patent- und Rechtsanwälte

(57) **Abstract**

Compression garment assembly set for a compression garment wrapping around a body part of a person to apply compression to the body part, wherein the compression garment assembly set comprises
- multiple bands for wrapping around the body part consecutively in a length direction of the body part, and
- for at least one pair of bands of the multiple bands, at least one band connector for releasably connecting abutting surfaces of the overlapping bands of the pair.

## Description

The invention concerns a compression garment assembly set for a compression garment for wrapping around a body part of a person to apply compression to the body part. The invention further concerns a compression garment and a method for assembling a compression garment.

Compression garments, such as wraps, bandages, stockings, socks and the like, are well-known in the art and serve to treat certain, for example vascular, diseases, improve physical performance and/or generally increase health and well-being of the person wearing them. For example, edema, in particular lymphedema, and various venous diseases may be treated using compression garments.

A compression garment exerts pressure inwardly on a person's body part, for example a limb. However, the body shapes of persons differ. Most compression garments do not allow for optimal fitting to each and every uniquely shaped body part. Furthermore, the locations where therapeutic compression is best applied to a body part and the strength of compression, in particular the compression profile, are unique for each patient. Hence, compression garments have been proposed which may be wrapped around the body part and provide adjustment options. For example, a compression garment may comprise multiple bands which are adjustable on their own. Furthermore, it has been proposed to provide individually manufactured garments customized for a specific person, for example a tubular elastic compression stocking with the material's stretch characteristics varying along the length of the compression garment to create different tensions.

Multi-part compression garments also contribute to better adjustability. For example, compression garments having a body portion and a spine portion have been proposed, as well as compression garments with attachable bands provided in addition to a body and/or a spine portion.

For example, US 7,867,185 B2 proposes a method of applying therapeutic compression to a patient's body limb, comprising measuring the dimensions of a patient's body limb; determining a desired compression profile for the body limb; and manufacturing a compression garment. The compression garment comprises an elongated body dimensioned to be wrapped around a length of a patient's limb to apply a therapeutic compression to the limb and a plurality of tension bands along the length of the elongated body. The tension bands are of different widths such that applying an equal tension to the bands results in different compressions being applied by the bands along the length of the patient's limb. The elongated body and the tension bands are manufactured based upon the measurements. However, an individual garment is produced, without any customization options for another patient or due to changes regarding the body limb.

EP 2 440 167 A1 discloses a compression garment for providing compressive force to a limb. The garment comprises a sleeve and multiple compression bands configured to extend about a portion of the sleeve and configured to impart a therapeutic compression to the limb. The bands are sewed to a spine. In EP 3 188 701 A1, a compression garment comprises a body dimensioned to wrap at least partially around a length of a limb of a subject. The body has an adjustable length. A plurality of trimmable tension bands is positioned along the adjustable length of the body. The garment further comprises an adjustable limb positioning sleeve positioned on the body and/or tension bands and a plurality of removable fasteners for attachment of the tension bands onto the body or tension bands.

EP 2 856 982 A1 discloses a compression binder consisting of multiple elastic bands that overlap and are attached to each other by small, vertical stitches that integrate into the material forming the compression binder and are arranged with an equal relatively short distance between them along the length of the elastic bands. Each elastic band has fabric hook and loop fasteners at first and second ends of the bands. The vertical stitch method of conjoining the elastic bands allows for individual compression adjustments of each elastic band which allows compression to be varied along a vertical line on the patient's abdomen so that compression can be adapted to the type of surgery performed and to the level of comfort desired by the patient.

However, such known compression garments are either customized for a certain patient, causing effort and additional cost, or do not provide enough flexibility to be adjusted for use with multiple different persons, in particular in body regions which often differ strongly from individual to individual. For example, differences in shape and length as well as regarding compression zones for a certain person may occur.

An example for such a body region is the head-neck-shoulder region, where subjects may have different neck shapes/tapering, different head and shoulder sizes, and different neck lengths. Compression to a body part, for example the neck, has to be applied consistently despite strongly varying shape/geometry in the target area. But also regarding other body regions and parts, for example limbs, there is a need for an option to flexibly and simply provide compression garments adjusted to the individual body part length, geometry and anatomy.

It is an object of the current invention to provide an option to flexibly and easily assemble compression garments adjusted to various features, including the length of the body part, of the person to wear it.

This object is achieved by providing a compression garment assembly set, a compression garment and a method according to the independent claims. Advantageous embodiments are described in the dependent claims.

A compression garment assembly set for a compression garment for wrapping around a body part of a person to apply compression to the body part, according to the invention, comprises:
- multiple bands for wrapping around the body part consecutively in a length direction of the body part, and
- for at least one, in particular at least for each lengthwise adjacently placeable, pair of bands of the multiple bands, at least one band connector for releasably connecting abutting surfaces of the overlapping bands of the pair.

Hence, it is proposed to provide at least two bands (which may also be called straps) and a band connector to be able to flexibly combine the bands along the length of the body part to form a garment adjusted to the geometry and anatomy of the body part of the person (subject). The band connector is placed between the bands of a pair to be attached together and has two opposing attachment surfaces on opposing sides, each attaching to another band. The band connector hence serves to form a garment, in particular having a length and/or shape adapted to the person and their compression needs. By refraining from the use of a common spine or a common body portion for all bands, flexibility regarding garment assembly is increased, since restrictions imposed by such a common spine or body portion do not exist. However, the band connectors are easy to handle and apply and allow easy assembly and donning of the garment, preferably even in the case of doffing and re-donning, as further discussed below.

It is noted that the band connector is an attachment device fixating the overlapping abutting surfaces of overlappingly placed bands together, and hence has its attachment surfaces on two opposing sides of the flat band connector, which preferably fully serve to provide attachment. On the other hand, a fastener, in particular a fastener element of the bands, acts to connect an end of a band elsewhere to close the bands when wrapped around the body part and provide compression. Such fastener elements usually prolong the band and comprise, if removable, attachment sections on one side of the fastener element.

The person may, for example, be a patient. An assembled compression garment may be used to treat edema, in particular lymphedema, venous diseases and/or muscular anomalies. The body part may be a limb, for example a leg and/or an arm, but may preferably comprise the neck and/or a part of the torso, for example the abdomen. In this description, the word "inner" refers to the side or direction towards the body part, while "outer" refers to the side or direction away from body part when the compression garment is worn.

The bands may be elastic or inelastic and/or directly apply compression. However, additional components of the garment may also be used to apply the compression, as further detailed below. Band materials may be selected based on desired characteristics, for example regarding their optical appearance, like color, patterns and prints. Characteristics like weight, heat and moisture management qualities, cooling effect, antimicrobial treatment, adhesion/surface friction properties and/or others, may also be chosen according to the application, as in principle known from the art. In particular, the bands may comprise a layer structure.

In embodiments, the band material may comprise a short-stretch breathoprene composite and/or nylon. In particular, such neoprene-like materials are also available having a hook-receptive loop surface. Hence, generally, the bands may comprise a loop surface, in particular on both their sides. This is particularly advantageous if the band connector comprises a hook material. For example, the loop surface may be a nylon-elastane loop surface. In a concrete example, the bands may comprise outer layers of nylon and/or elastin and/or any breathable material and a loop-surface backing, and an open-celled foam in between. In embodiments, the bands may comprise a velour material, for example to improve wearing comfort. The bands may be contiguous material pieces and/or be cut from a material sheet, in particular if trimmable.

As already mentioned, the band connector is preferably flat, such that the bands of the pair may closely abut each other in the overlapping areas and no relevant cavities are created. The band connector may preferably be one-piece for easy handling. For example, the band connector may have a thickness of 0.5 to 3 mm perpendicular to the attachment surfaces.

According to the invention, one band connector is provided to connect exactly two bands, namely a pair of bands. By connecting the bands in a pairwise manner using the band connectors, maximum flexibility is provided, in particular in comparison to a single spine element of a given length or a body portion of a given length. The length and/or the shape of the garment can thus be adapted easily as required for the individual person. Various combinations of bands along the length of the compression garment/body part can be implemented with varying overlaps and/or relative orientations and/or individual band shapes. In particular, regarding shape, bands of the pair may be releasably fixated onto each other in different orientations.

In particular, bands may have different compression characteristics or may be adjusted to apply different compression, allowing to also adapt compression profiles along the length of the garment and/or in particular also in a circumferential direction.

To connect a pair of bands, the band connector may be correspondingly sized, for example having a width of less than one half, in particular less than one third, of the width of the narrowest of the bands in the length direction of the body part. To, however, provide a sufficient attachment area, in a direction perpendicular to the width of the band connector, the band connector may have a larger extension, for example up to the width of the narrowest of the bands. The band connector may, for example, have a rectangular shape and/or an elliptical shape. Generally speaking, the minimal extension of the attachment surfaces of the band connector may, for example, be 0.5 to 2 cm and/or the maximum extension of the attachment surfaces of the band connector may be, for example 1.5 to 5 cm.

Preferably, the band connector comprises at least one fastener material to establish the releasable connections. In particular, the fastener material may connect to a mating fastener material of the respective band. Preferably, the whole surface of the bands, in particular on both sides, is made from a mating fastener material, in particular a loop material. In other words, and generally speaking, the fastener may comprise a male fastener material mating with a female fastener material of the band of the pair.

Preferably, the band connector comprises a loop material and/or, preferably, a hook material to releasably attach to a hook material and/or a loop material, respectively, of the bands. In especially preferred embodiments, the attachment surfaces of the band connector may comprise a hook material connecting to a loop material of the bands, in particular covering at least essentially the whole surface of the bands. In other embodiments, a mixed loop and hook material may be used for the band connector, able to attach to itself or other pure hook or loop surfaces.

In other embodiments, the fastener material may also comprise at least one adhesive. For example, the band connector may comprise a re-usable, in particular two-sided, adhesive tape. Other options for fasteners usable in the band connector comprise snaps, magnetic fasteners and the like.

While it is, in principle, conceivable that the band connector is permanently fixed to one of the bands, for example sewed to the band, in preferred embodiments the band connector is releasably attachable to each band. Hence, the band connector may be placed where needed, be used for different pairs of bands or even between a band and another component, as further discussed below.

In other words, having the band connector being releasably connectable to both bands of the pair allows to use the band connector flexibly for different bands, such that, for example the order of the bands along the length of the body part or the bands constituting the compression garments may be freely chosen. Furthermore, the band connector may flexibly be positioned between the bands of the pair, in particular depending on the overlap to be provided and/or such that, in the overlapping region, the bands are connected at an edge of the outer band, such that the bands tightly abut at the edge and the edges to not protrude. In particular, the band connector may also be placed as a marker, for example to indicate the ideal position of the edge of the other, outer band of the pair. The band connector may be placed in different orientations, for example following the orientation of one of the bands, in particular the outer band.

In this context, in particularly advantageous embodiments, the band connector may comprise two sides each releasably connectable to a band of the pair, wherein a first side is configured to provide stronger fixation to the respective band than the other, second side. As explained above, the sides are opposite, facing in different directions of the band connector. In the worn state of the compression garment, one, in particular the first, side faces towards the body part, while the other, in particular the second, side faces outwards away from the body part. In this manner, one connection at the first side can be considered semi-permanent, for example such that it will be the connection remaining when the garment is doffed. The connection to the second side is an "easy-to-release"-type connection, allowing easy donning and doffing. The band connector, due to the semi-permanent connection at the first side, remains in place when the band from the second side is removed. Hence, there is no need to re-place the band connector if the garment is to be donned again. In addition, the band connector may serve as a marker, for example to indicate the position of the edge of the band to be fastened to the second side and/or its orientation. In summary, the band connector stays in place, allowing easier re-donning. Generally put, the stronger first side may be connected to a "host band" or generally "host component", where repositioning of the band connector placement is required less frequently, leaving the weaker second side exposed to attach to the other band as a component that is frequently adjusted, opened/closed and so on.

The higher connection strength at the first side may, for example, be provided by having a different hook type than the hook type of the second side or a higher density of hooks in the hook material. In another example, an adhesive backed hook and/or loop material may be used, which typically provides a stronger bond on the adhesive side than on the hook and/or loop side.

In preferred embodiments, the compression garment assembly set may further comprise at least one securing strip for applying over an edge on the outside of the pair of bands attached to each other by the band connector. The securing strip may, for example, be provided in another color than the band connector to be clearly distinguishable. In particular, the securing strip may comprise fastener material also used on the band connector on one side, in particular the second side, and no fastener material on the other, outer side. The fastener material, for example hook material, may connect to a mating fastener material on the outside of the bands, for example loop material. The securing strip may, for example, be used on the finally donned compression garment where needed for additional stability and/or wearing comfort. It may also be used to connect two bands which do not overlap, but abut in the length direction of the body part.

In advantageous embodiments, the bands may be trimmable and/or comprise at least one fastener element, in particular releasably, attachable to one end of a respective band of the plurality of bands and comprising a fastening section to releasably attach along the outside of the same band for fastening the band around the body part. In particular, the bands may be made of one piece of uniform material to be trimmable in each dimension. For example, a band may be manually held roughly in position at the body part, marked and trimmed, in particular in its longitudinal direction, being the circumferential direction of the body part. To hold the bands in position when the band is wrapped around the body part, in particular to apply compression, fastener elements are provided. In particular, after trimming the bands regarding their lengths, a fastener element may be attached to one of the ends of the remaining band length, to be able to attach the band onto itself and/or another band to hold it in position when the garment is donned, as in principle known from the art. The fastener element preferably has the width of the respective band and may comprise hook material at at least one side, in particular the inner side, to attach to loop material on the outer side of the band. The fastener element may be shaped to, for example, become narrower/taper away from the end of the band to indicate the presence of a fastener.

In a first concrete embodiment, the fastener element may comprise two sections each releasably connectable to the band, wherein a first section to be attached to the end of the band is configured to provide stronger fixation to the respective band than the other, second section, which may in particular be arbitrarily placed along the outside of the band. Preferably, both sections are provided, in particular separated and/or distinguishable, on one side, namely the inner side when worn, of the fastener element. For example, a tapering shape may indicate the second section to be placed away from the end of the band. However, other embodiments having the first and the second sections on different, opposing sides are also conceivable, although less preferred. Using such a configuration having a stronger attachment to the end of the band, the fastener element remains attached to the end of the band even if the outer surface attachment of the second section is unfastened.

In a second concrete embodiment, the compression garment assembly set may further comprise at least one intermediary element for each fastener element, the intermediary element comprising a first attachment section to releasably attach the intermediary element to the end of the band and a second attachment section to releasably attach the fastener element to the intermediary element, wherein the releasable attachment of the attachment sections is stronger than the releasable attachment of the fastener element to the band. The first and second attachment section of the intermediate element, which may provide the same attachment strength, are preferably provided on the same side of the intermediate element, in particular the inner side when the compression garment is worn. This second concrete embodiment allows the fastener to be fabricated with, for example, the same attachment strength over its surface, allowing easier installation regardless of its orientation. As the first concrete embodiment, this second concrete embodiment also allows the fastener element to remain attached to the end of the band even if the fastening element is unfastened from elsewhere on the same and/or another band, for example when doffing the compression garment.

Preferably, the compression garment assembly set may comprise bands of different width. In this manner, bands of different widths with different overlaps can be combined to create compression garments of different lengths along the body part and/or with differently sized compression zones in length direction of the body part. In particular, each band or combination of bands may define a compression zone of a compression profile.

In this context, but also generally, it is especially advantageous to provide at least one of the bands curved to conform to a geometry of the body part. In particular, the body part may have an increasing or decreasing extension along the length direction of the body part, i.e., be tapered. In particular, the band may conform to an at least essentially conical geometry. The curvature of the band may be symmetrical. For example, at least one curved band may comprise two straight sections joined by a central curve section. The straight sections may, for example, enclose an angle of 100 to 150 degrees. If such a band is wrapped around a body part, it assumes a conical shape, which can be adjusted to a tapered section of the body part. Likewise, such bands can adapt to areas with shelves. In particular, since, in the current compression garment assembly set, bands may be individually chosen and combined, providing bands of different curvature and/or widths and/or integrating them individually in the garment allows optimal fit to the body part of a certain person.

In this respect, other symmetric or asymmetric geometries of at least one band of the multiple bands may be provided or created, e.g., via trimming, to improve the band fit to contours of the body part and/or to adjust the coverage area.

Generally speaking, the compression garment assembly set may comprise bands having different geometries, in particular at least one curved band and/or at least one straight band and/or bands of different widths. These bands can be combined using the at least one band connector in various ways in the length direction of the body part. Each band provides a garment section of a shape according to its geometry, for example a conical shape for a curved band. In this manner, further flexibility in building the concrete garment is provided. In particular, the garment and/or compression zones can be perfectly adapted to concrete shapes of the body part.

It is noted that a band may be used in different orientations. For example, a curved band may be applied to the head tapering to smaller extensions in the neck area as well as to the shoulders narrowing to the neck.

In a concrete embodiment for application in the neck area of the body, for example to the neck itself as the body part, the plurality of bands may comprise
- a neck band for application to a neck area of the person, and
- a chin band for application to a chin area of the person,
- wherein the neck band has a larger width than the chin band.

Preferably, in use, the band connector may be used to attach the chin band to the outside of the neck band. The neck band may, for example, have a width of 9 to 12 cm, for example 10.5 cm, and/or the chin band may have a width of 6 to 9 cm, for example 7.5 cm. Both the neck band and the chin band are preferably curved, particular with an angle of 100 to 150 degrees, to conform to the corresponding body part geometries. For example, to assemble a compression garment, the neck band may first be optionally trimmed and provided with a fastener element around the neck. Thereafter, the chin band may be optionally trimmed and provided with a fastener element. The chin band may be positioned with a certain overlap relative to the neck band according to the body part geometry and attached with its inner side to the outside of the neck band, for example at a central position at the back of the head. The curved neck band may be used in both orientations, for example in the shoulder area versus the upper neck area.

In embodiments, the plurality of bands may further, in particular in addition to the curved neck and chin bands, comprise a straight band, in particular having a width in between the widths of the neck band and the chin band. Such a straight band may be used for a relatively straight neck having little or no taper, in particular also as a central band attached to the neck band in the shoulder area and the chin band in the chin area.

Preferably, the compression garment assembly set may further comprise at least one compression pad to apply compression to the body part, wherein the bands are configured to fixate the compression pad against the body part. Such compression pads are already known in the art and are an advantageous addition to the compression garment assembly set, providing a component for optimal distribution of compression. To be better adaptable to the geometry of the body part, the compression pad may be trimmable.

In an especially preferred embodiment, the compression pad and the band connector may be configured to also attach one band to the compression pad. Hence, at least one of the band connectors may also be used to attach a band to an additional component of the garment, in particular a compression pad, in particular at different locations to allow flexible adjustment to the body part and an area to be treated. An additional use for band connectors is hence provided.

In particular, the outer surface, which is positioned away from the body part, of the compression pad may comprise a mating fastener material mating with the fastener material of the band connector, in particular the same mating fastener material as the bands. The mating fastener material may, again, be a loop material, connecting with a hook material of the band connector.

This may preferably be combined with the band connector having two sides of different connection strength, as, even when the compression garment is doffed, the band connectors may remain in place, either on the compression pad or the band, allowing easy and exact reconnection. However, the compression pad as a whole can also "semi-permanently" stay attached to the band, in particular when doffed.

In this context, an additional pad connector may be conceivable, for example having two strongly fastening sides.

Preferably, the compression pad may comprise an inner surface to be applied towards the body part, wherein the inner surface is textured. While the inner surface may be flat, it preferably has a patterned and/or irregular textured surface for distributing compression to the body part. Textured surfaces are useful for creating local high-low pressure gradients. These gradients can help move edema and break down tissue due to the massaging effect, in particular in the case of scar tissue, where adhesions and/or collagen fibers may be broken down. Preferably, the surface may be textured in a pattern of protruding regular hexagons. The raised hexagon pattern distributes higher compression into localized areas where edema is present or where more focused pressure is desired.

In embodiments, the compression pad may have a layered structure. For example, the pad may comprise of a thermoformed three-layer composite, in particular with the texture, for example hexagonal protrusions, on the inner surface of the innermost layer. This innermost layer may consist of a material also having a heat management function, in particular a cooling effect. Such a material may, for example, be a warp knit material with a mineral treatment that provides a cooling effect for heat management. An example is the brrr° (registered trademark) material available from brrr°, Atlanta, GA, United States. The central layer may consist of a soft open-cell foam and the outermost layer may consist of a nylon loop material receptive to a hook material of band connector. The loop material allows the compression pad to be connected to the bands via the double-sided hook material band connector.

In preferred embodiments, the compression garment assembly set may comprise at least one intermediate component connectable to a band at opposing edge regions of the intermediate component by band connectors. That is, the intermediate component may, in other words, provide a prolonged band. Band ends of bands are connected in both opposing edge regions to the intermediate component. For example, a compression pad and/or another band may serve as such an intermediate component.

In particular, the compression pad or generally intermediate component may be dimensioned to attach one band at each of the opposing edge regions. For example, the intermediary component may be associated with bands of a predetermined width, wherein the extension of the opposing edges is less than two times the predetermined width, in particular less than 1.5 times the predetermined width, such that the intermediate components and the bands attached to the opposing sides form a complete band structure for wrapping around the body part.

It is noted that, in particular in cases in which all or at least most of the components of the compression garment assembly set are also part of the assembled garment, the compression garment assembly set may itself be understood as an, initially, disassembled compression garment which may be configured and adjusted to fit a specific body part by using the degrees of freedom provided during assembly, in particular by pairwise attachment of bands to each other without any restricting main component. Here, the band connector is used to pairwise connect bands with desired overlap and/or relative orientation, in particular in a desired order of bands in the length direction of the body part. Such compression garment assembly sets (or kits) may also be termed "reduction kits". However, in any case, the invention also encompasses a compression garment assembled from a compression garment assembly set according to the invention.

The invention further concerns a method for assembling a compression garment for wrapping around a body part of a person to apply compression to the body part, wherein
- multiple bands for wrapping around the body part consecutively in a length direction of the body part are provided, and
- for at least one pair of bands, abutting surfaces of the overlapping bands of the pair are releasably connected using at least one band connector.

In the method for assembling a compression garment, a compression garment assembly set as described above may be used. All features and remarks regarding the compression garment assembly set analogously apply to the method for assembling a compression garment and vice versa.

In particular, bands to be used for the compression garment may be chosen from the plurality of bands. The bands may be roughly fitted to their relative location on the body part by trimming and adding a fastener element. The bands may be pairwise connected using a band connector per pair, in particular successively in a length direction of the body part.

In the concrete example of the head-neck compression garment assembly set or head-neck compression garment ("head-neck reduction kit"), it may first be decided, if a straight band is provided in addition to the curved neck band and the curved chin band, which combination of bands is used to assemble the compression garment for a specific person. For example, all three bands may be employed, with the neck band used in the lower neck area to also conform to the transition to the shoulders, the straight band used for the neck and the chin band in the chin area at the lower head. Alternatively, two bands may be used, depending on the shape and length of the neck and the treatment area, for example the neck band and the chin band or the straight band and the chin band.

If the head-neck compression garment comprises a compression pad, the compression pad may first be trimmed and configured for use in the heck-neck treatment area. Then, the bands used may be customized to the specific person by wrapping them around the section of the body part where they should be applied, optionally trimming in a longitudinal direction of the band, and adding a fastener element. Finally, each pair of two bands adjacent and overlapping in length direction are attached to each other using at least one, in particular one or two, band connectors in the overlap region, in particular with the first side providing a stronger connection force oriented towards the inner of the two overlapping bands, simplifying donning and doffing.

Further details and advantages of the invention become apparent from the following description of concrete embodiments, taking in conjunction with the drawingw, which show:
- Fig. 1: schematically a general embodiment of a compression garment assembly set according to the invention,
- Fig. 2: a first embodiment of a band,
- Fig. 3: the first embodiment of the band in a closed view for application to a person,
- Fig. 4: a second embodiment of a band,
- Fig. 5: the second embodiment of the band in a closed view for application to a person,
- Fig. 6: a third embodiment of a band,
- Fig. 7: the third embodiment of the band in a closed view for application to a person,
- Fig. 8: a side view of a fastener element,
- Fig. 9: an alternative configuration for attaching a fastener element,
- Fig. 10: a side view of a band connector,
- Fig. 11: the application of the band connector to connect a pair of bands,
- Fig. 12: a perspective view of a compression pad,
- Fig. 13: a schematical side view of the compression pad,
- Fig. 14: the application of the band connector to attach the compression pad to a band,
- Fig. 15: a head-neck compression garment assembly set according to the invention,
- Fig. 16: a compression garment assembled from the head-neck compression garment assembly set of Fig. 15,
- Fig. 17: the compression garment of Fig. 16 in a partly disassembled, doffed state,
- Fig. 18: the compression garment of Fig. 16 and 17 in a worn state in a side view,
- Fig. 19: a second compression garment assembled from the head-neck compression garment assembly set of Fig. 15 in a side view,
- Fig. 20: an embodiment having an intermediary component in a band,
- Fig. 21: another configuration to attach a compression pad to bands,
- Fig. 22: a securing strip in a side view, and
- Fig. 23: the use of a securing strip to connect lengthwise abutting bands.

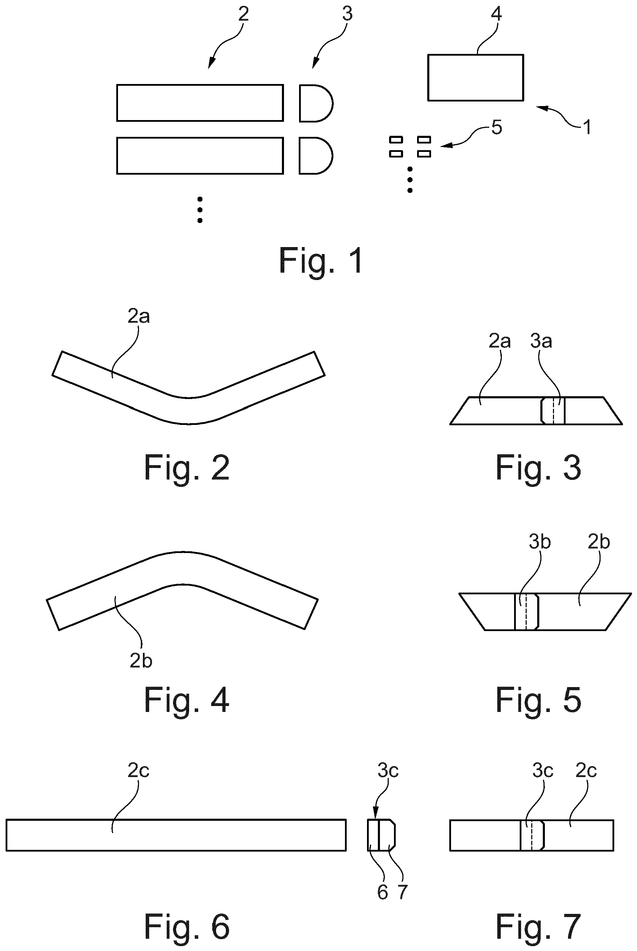

Fig. 1 schematically shows general components of a compression garment assembly set 1 according to the invention. The compression garment assembly set 1 comprises a plurality of bands 2, fastener elements 3 associated with each band 2, a compression pad 4, which may be associated with at least one of the bands 2, and multiple band connectors 5 for releasably connecting abutting surfaces of the overlapping bands 2 of a pair of bands 2. The compression pad 4 may be used as an intermediary component. Optional further components (not shown) comprise further intermediary components as well as securing strips.

Generally speaking, the bands 2 may have different geometries, for example be at least partly curved, and/or may have different widths. Since they are pairwise releasably connectable in length direction of a body part to be treated, the bands 2 may be flexibly chosen and combined as required to customize an assembled compression garment to the concrete geometry and anatomy of the body part. In particular, bands 2 may also have different compression characteristics and/or provide different compression depending on how they are fastened when wrapped around the body part, thus also allowing to provide customized compression profiles.

The bands 2 may be at least partly elastic and/or at least partly inelastic. In a concrete embodiment, the bands 2 are made from a short-stretch breathoprene composite with a nylon-elastane loop surface receptive to hook material on both sides of the material and an open-celled foam in between. This allows the band connector 5 as a double-sided hook material component to connect to the inner and outer band surfaces and the fastener elements 3 as closure hook tabs to connect to the outer surface of the bands 2. The bands 2 are cut from a uniform, contiguous piece of band material allowing them to be trimmed along any dimension, in particular along the length of the bands 2. In this manner, the bands 2 can be fit to the circumference of the body part and fastener elements 3 can be added to a free end of the bands 2. In other embodiments, the bands 2 may also comprise velour material.

Fig. 2 shows a first embodiment of a band 2a. The band 2a is curved and has a width of, in this example, 7.5 cm. When wrapped around the body part, it adapts a conical shape due to the curvature, as shown in Fig. 3, where also the fastener element 3a is shown, allowing to attach the end of the band 2a anywhere onto itself to close the band 2 in position around the body part, applying compression.

Fig. 4 shows a second embodiment of a band 2b. The band 2b is also curved and has a width of, in this example, 10.5 cm. It is shown in another orientation to clarify that curved bands 2a, 2b may be used both for enlarging and narrowing sections of the body part, depending on the orientation. The conical shape of band 2b when wrapped around the body part is shown in Fig. 5, where also the fastener element 3b is shown analogously to Fig. 3.

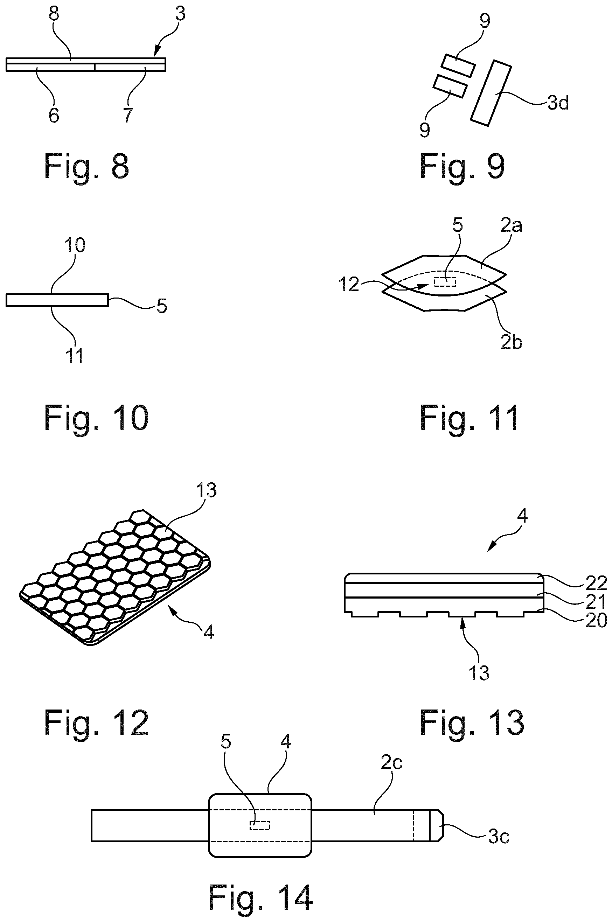

Fig. 6 shows a third embodiment of a band 2c. The band 2a is straight and has a width of, in this example, 8 cm. The fastener element 3c is also shown in Fig. 6 in its non-attached version and will be discussed later with respect to additional Fig. 8. When wrapped around the body part, the band 2c adapts a straight shape, as shown in Fig. 7.

Fig. 8 is a schematical side view of fastener element 3a. Fastener elements 3a and 3b are configured correspondingly. As can also be seen from Fig. 6 and indicated in Fig. 3, 5 and 7, the fastener elements 3a, 3b and 3c comprise two sections 6, 7 each releasably connectable to the respective band 2a, 2b, 2c by comprising hook material, wherein a first section 6 to be attached to the end of the respective band 2a, 2b, 2c is configured to provide stronger fixation to the respective band 2a, 2b, 2c than the other, second section 7. Both sections 6, 7 are provided on the same, inner side of the fastener element 3a, 3b, 3c, which thus forms a split attachment surface. There may be a separating section between the two sections 6, 7. The other, outer side 8 has no fastening characteristics. However, embodiments may be conceivable where the sections 6, 7 are provided on different sides of the fastener element 3a, 3b, 3c.

By having the section 6 providing a stronger connection force, the connection at the end of the respective band 2a, 2b, 2c remains in effect even if the less strong connection at the section 7 is released, for example when doffing the compression garment.

Fig. 9 shows another approach to strongly semi-permanently fasten a fastener element 3d to the end of a band 2. Here, two intermediate elements 9 are used, which each comprise a first attachment section to releasably attach the intermediary element 9 to the end of the band 2 and a second attachment section to releasably attach the fastener element 3d to the intermediary element 9. The releasable attachment of the attachment sections of the intermediary element 9 is stronger than the releasable attachment of the fastener element 3d to the outside of the band 2.

Turning to Fig. 10, the band connectors 5 of the compression garment assembly set 1 are, in this embodiment, flat and at least essentially rectangular. To be able to accommodate as many overlap lengths as possible, the smaller extension (preferably placed along the width of the bands 2, when applied) of the outer and the inner sides 10, 11 of the band connectors is less than one third of the smallest width among the bands. To provide the required connection strength, the maximum extension may be larger than the minimal extension, for example two or three times the minimal extension. In other embodiments, the band connectors 5 may also be elliptical in shape.

The band connector 5 has two opposing attachment surfaces on the opposing first and second sides 10 and 11. In this embodiment, each attachment surface comprises hooks to connect with the loops of the bands 2. The band connector 5 is placed between bands 2 to pairwise connect and form garment sections. The attachment surfaces on sides 10, 11 may provide different connection strengths and/or comprise different fastener types.

In the embodiments shown here, the first side 10 is configured to provide stronger fixation to the respective band than the other, second side 11. That is, the first side 10, which is preferably applied to the outside of the inner of the overlapping bands 2, provides a semi-permanent connection and remains in place when the connection to the second side 11 is released, providing ease of handling when donning and doffing the compression garment. Generally speaking, the stronger connecting side 10 may be connected to a band 2 where repositioning is required less frequently, leaving the weaker strength connecting side 11 exposed to attach to the band 2 that is frequently adjusted, opened and closed.

The band connector 5 may be made of nylon, spandex, silicone or the like and comprise hook material as fastener material mating to the mating fastener material, namely loop material, of the bands 2.

Fig. 11 illustrates the use of the band connector 5 to flexibly connect a pair of bands 2, in this case exemplarily the two bands 2a, 2b. As can be seen, the band connector 5 is placed between the bands 2a, 2b in the overlapping region 12. It may preferably be positioned to indicate the desired position of an edge of the outer band 2a.

Fig. 12 shows a perspective view of the compression pad 4. On the visible inner side, the compression pad 4 comprises a textured inner surface 13 to be applied in a treatment area of the body part. In this example, the textured inner surface 13 comprises hexagonal protrusions. However, other configurations having a pattern or being irregular are also possible.

In a concrete embodiment, as shown in Fig. 13, the compression pad 5 may consist of a thermoformed three-layer composite with a raised hexagonal pattern. The raised hexagonal pattern distributes higher compression into localized areas where, for example, edema is present or where more focused pressure is desired. The inner layer 20 consists of a warp knit material with a mineral treatment that provides a cooling effect for heat management. The middle layer 21 consists of a soft open-cell foam and the outer (base) layer 22 consists of a nylon loop material receptive to hook material of the band connectors 5.

As shown in Fig. 14, the loop material of the outer layer of the compression pad 5 allows the compression pad 5 to be connected to a band 2, in Fig. 14 exemplarily the band 2c, via a band connector 5. The band 2c then fully encompasses the compression pad length.

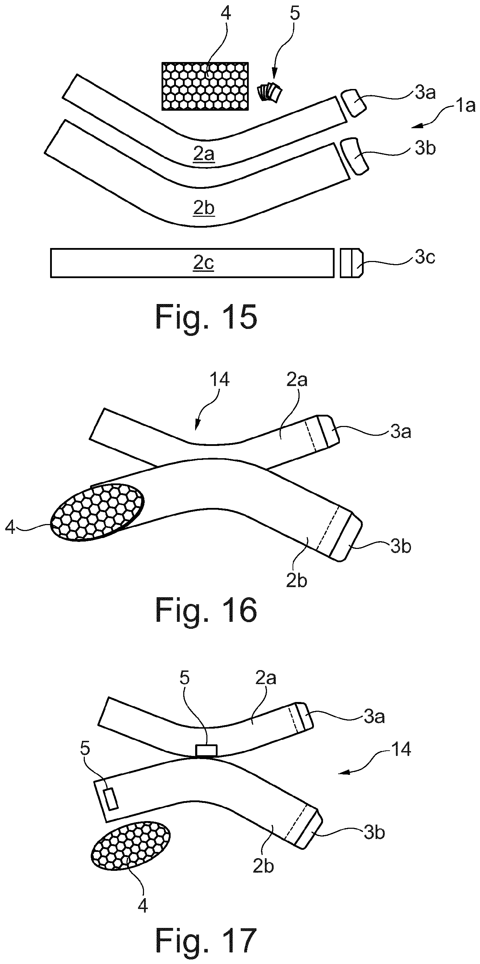

Fig. 15 shows components of a head-neck compression garment assembly set 1a as a concrete exemplary embodiment. The assembly set 1a comprises the compression pad 4, the band 2a as a curved chin band, the band 2b as a curved neck band, the band 2c as a straight band, the fastener elements 3a, 3b and 3c associated with the respective bands 2a, 2b and 2c, and multiple band connectors 5. The bands 2a, 2b, and 2c can be combined to assemble a compression garment depending on the body part and the treatment area in various ways.

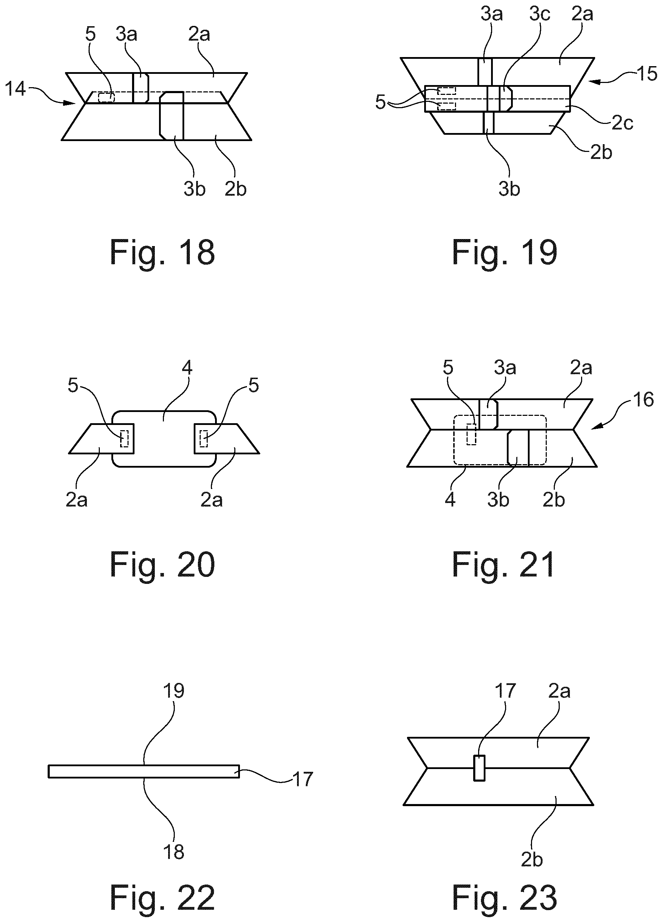

Figs. 16 to 18 show a first compression garment 14, wherein the broader curved neck band 2b is used for the transition area of the shoulders to the (short) neck and the chin band 2a is used for the chin region. Since the treatment region is at the neck, the (correspondingly trimmed and thus customized) compression pad 5 is attached to the neck band 2b via a first band connector 5. The pair of neck band 2b and chin band 2a is connected in an overlap region by a second band connector 5. The band connectors 5 can be seen in the partially disassembled view of Fig. 17. Fig. 18 shows the resulting shape. For simplification, the compression pad 4 and its connector 5 are not shown in Fig. 18.

Fig. 19 shows the shape of a second assembled compression garment 15 also employing the straight band 2c. As can be seen, the neck band 2b and the straight band 2c form a first pair connected by a first band connector 5, and the straight band 2c and the chin band 2a form a second pair connected by a second band connector 5. While not shown in Fig. 19, the straight band 2c may, of course, span a distance between the curved bands 2a, 2b. Fig. 19 also nicely illustrates the formation of garment sections contributing to a compression profile along the body part. For example, all bands 2a, 2b, 2c defining different garment sections may apply different compression.

Of course, compression garments may also be provided for other body parts, for example limbs, and in particular in regions having a difficult, varying geometry to adapt to.

Fig. 20 exemplarily shows the use of the compression pad 4 as an intermediary component. As can be seen, a band 2a is attached with an end in opposing edge regions of the compression pad using band connectors 5. In principle, also fastener elements 3a could be used here. Other intermediary components are conceivable, for example to prolong a band 2.

Fig. 21 shows a configuration of a compression garment 16 in which the compression pad 4 is attached to both bands 2a and 2b using a single band connector 5. For example, the band connector 5 may connect the abutting bands 2a, 2b via the compression pad 4.

Fig. 22 shows a securing strip 17, which may be part of the compression garment assembly set 1, 1a. The securing strip 17 comprises on inner side 18 having a fastener material, here a hook material, and an opposing outer side 19 without fastening characteristics. The securing strip 17 may be used to additionally secure a pair of bands 2 connected by a band connector 5 together, but may also be used to attach bands 2a, 2b having no overlapping region, but abutting in length direction of the body part, as shown exemplarily in Fig. 23.

## Claims

1. Compression garment assembly set for a compression garment wrapping around a body part of a person to apply compression to the body part, wherein the compression garment assembly set comprises
- multiple bands for wrapping around the body part consecutively in a length direction of the body part, and
- for at least one pair of bands of the multiple bands, at least one band connector for releasably connecting abutting surfaces of the overlapping bands of the pair.

2. Compression garment assembly set according to claim 1, wherein the band connector comprises a loop material and/or a hook material to releasably attach to a hook material and/or a loop material, respectively, of the bands.

3. Compression garment assembly set according to claim 1 or 2, wherein the band connector is releasably attachable to each band.

4. Compression garment assembly set according to claim 3, wherein the band connector comprises two sides each releasably connectable to a band of the pair, wherein a first side is configured to provide stronger fixation to the respective band than the other, second side.

5. Compression garment assembly set according to one of the preceding claims, wherein the compression garment assembly set further comprises at least one securing strip for applying over an edge on the outside of the pair of bands attached to each other by the band connector.

6. Compression garment assembly set according to one of the preceding claims, wherein the bands are trimmable and/or comprise at least one fastener element attachable to one end of a respective band of the plurality of bands and comprising a fastening section to releasably attach along the outside of the same band for fastening the band around the body part.

7. Compression garment assembly set according to claim 6, wherein
- the fastener element comprises two sections each releasably connectable to the band, wherein a first section to be attached to the end is configured to provide stronger fixation to the respective band than the other, second section, or
- the compression garment assembly set further comprises at least one intermediary element for each fastener element, the intermediary element comprising a first attachment section to releasably attach the intermediary element to the end of the band and a second attachment section to releasably attach the fastener element to the intermediary element, wherein the releasable attachment of the attachment sections of the intermediary element is stronger than the releasable attachment of the fastener element to the band.

8. Compression garment assembly set according to one of the preceding claims, wherein the compression garment assembly set comprises bands of different width and/or at least one of the bands is curved to conform to a geometry of the body part.

9. Compression garment assembly set according to claim 8, wherein the plurality of bands comprises
- a neck band for application to a neck area of the person, and
- a chin band for application to a chin area of the person,
- wherein the neck band has a larger width than the chin band.

10. Compression garment assembly set according to claim 9, wherein the plurality of bands further comprises a straight band, in particular having a width in between the widths of the neck band and the chin band.

11. Compression garment assembly set according to one of the preceding claims, wherein the compression garment assembly further comprises at least one compression pad to apply compression to the body part, wherein the bands are configured to fixate the compression pad against the body part.

12. Compression garment assembly set according to claim 11, wherein the compression pad is trimmable and/or the compression pad and the band connector are configured to also attach one band to the compression pad.

13. Compression garment assembly set according to claim 11 or 12, wherein the compression pad comprises an inner surface to be applied toward the body part, wherein the inner surface is textured.

14. Compression garment assembled from a compression garment assembly set according to one of the preceding claims.

15. Method for assembling a compression garment wrapping around a body part of a person to apply compression to the body part, wherein
- multiple bands for wrapping around the body part consecutively in a length direction of the body part are provided, and
- for at least one pair of bands, abutting surfaces of the overlapping bands of the pair are releasably connected using at least one band connector.
